# EUROPEAN PATENT SPECIFICATION

(11) **EP 1 119 548 B1**
(45) Date of publication and mention of the grant of the patent: **08.12.2004**
(21) Application number: 99947744.1
(22) Date of filing: 05.10.1999
(51) Int. Cl.: C07D 207/44, C07D 403/04, C07D 401/04, A61K 31/4015, A61P 25/28

(54) **3-(3-CHLORO-4-HYDROXYPHENYLAMINO)-4-(2-NITROPHENYL)-1H-PYRROLE-2,5-DIONE AS GLYCOGEN SYNTHASE KINASE-3 INHIBITOR (GSK-3)**
3-(3-CHLORO-4-HYDROXYPHENYLAMINO)-4-(2-NITROPHENYL)-1H-PYRROL-2,5-DION ALS GLYKOGEN SYNTHASE KINASE-3 INHIBITOR (GSK-3)
3-(3-CHLORO-4-HYDROXYPHENYLAMINO)-4-(2-NITROPHENYL)-1H-PYRROLE-2,5-DIONE UTILE COMME INHIBITEUR DE GLYCOGEN SYNTHASE KINASE-3 (GSK-3)

(30) Priority: 08.10.1998 GB 9821974; 14.12.1998 GB 9827521; 17.12.1998 GB 9827883; 10.03.1999 GB 9905518; 26.03.1999 GB 9907086; 16.08.1999 GB 9919362
(43) Date of publication of application: 01.08.2001
(73) Proprietor: SmithKline Beecham plc, Brentford, Middlesex TW8 9GS (GB)
(72) Inventor: COGHLAN, Matthew, Paul, SmithKline Beecham Pharma., Harlow, Essex CM19 5AW (GB); FENWICK, Ashley, Edward, SmithKline Beecham Pharma, Harlow, Essex CM19 5AW (GB); HAIGH, David, SmithKline Beecham Pharmac., Harlow, Essex CM19 5AW (GB); HOLDER, Julie, Caroline, SmithKline Beecham Pharma, Harlow, Essex CM19 5AW (GB); IFE, Robert, John, SmithKline Beecham Pharma., Harlow, Essex CM19 5AW (GB); REITH, Alastair, David, SmithKline Beecham Pharma., Harlow, Essex CM19 5AW (GB); SMITH, David, Glynn, SmithKline Beecham Pharma., Harlow, Essex CM19 5AW (GB); WARD, Robert, William, SmithKline Beecham Pharma., Harlow, Essex CM19 5AW (GB)
(74) Representative: Rutter, Keith, Dr.
(86) International application number: PCT/GB1999/003280
(87) International publication number: WO 2000/021927

(56) References cited:
- EP-A- 0 328 026
- WO-A-00/06564
- WO-A-97/41854
- WO-A-98/11104
- WO-A-98/16528
- WO-A-99/57117
- DE-A- 4 005 969
- DE-A- 4 005 970
- US-A- 3 335 147
- DATABASE CHEMABS [Online] CHEMICAL ABSTRACTS SERVICE, COLUMBUS, OHIO, US ZHANG, GUO-LIN ET AL: "Alkaloids from Hypecoum leptocarpum" retrieved from STN Database accession no. 124:82090 XP002135369 & PHYTOCHEMISTRY (1995), 40(6), 1813-16 ,

## Description

This invention relates to a novel method for the treatment and/or prophylaxis of conditions associated with a need for inhibition of glycogen synthase kinase-3 (GSK-3), especially diabetes, including chronic neurodegenerative conditions, including dementias such as Alzheimer's disease, neurotraumatic diseases, such as acute stroke, mood disorders such as schizophrenia and manic depression, and for the treatment and/or prophylaxis of hair loss and cancer, and to certain novel inhibitors of GSK-3 for use in such a method.

GSK-3 is a serine/threonine protein kinase composed of two isoforms (α and β) which are encoded by distinct genes. GSK-3 is one of several protein kinases which phosphorylates glycogen synthase (GS) (Embi *et al* Eur. J. Biochem. **(107)** 519-527 (1980)). The α and β isoforms have a monomeric structure of 49 and 47kD respectively and are both found in mammalian cells. Both isoforms phosphorylate muscle glycogen synthase (Cross *et al* Biochemical Journal (**303**) 21-26 (1994)) and these two isoforms show good homology between species (e.g. human and rabbit GSK-3α are 96% identical).

Type II diabetes (or Non-Insulin Dependent Diabetes Mellitus, NIDDM) is a multifactorial disease. Hyperglycaemia is due to insulin resistance in the liver, muscle and other tissues coupled with inadequate or defective secretion of insulin from pancreatic islets. Skeletal muscle is the major site for insulin-stimulated glucose uptake and in this tissue, glucose removed from the circulation is either metabolised through glycolysis and the TCA cycle, or stored as glycogen. Muscle glycogen deposition plays the more important role in glucose homeostasis and Type II diabetic subjects have defective muscle glycogen storage.

The stimulation of glycogen synthesis by insulin in skeletal muscle results from the dephosphorylation and activation of glycogen synthase (Villar-Palasi C. and Larner J. Biochim. Biophys. Acta **(39)** 171-173 (1960), Parker P J *et al*., Eur. J. Biochem. (**130**) 227-234 (1983), and Cohen P. Biochem. Soc. Trans. (**21**) 555-567 (1993)). The phosphorylation and dephosphorylation of GS are mediated by specific kinases and phosphatases. GSK-3 is responsible for phosphorylation and deactivation of GS, while glycogen bound protein phosphatase 1 (PP1G) dephosphorylates and activates GS. Insulin both inactivates GSK-3 and activates PP1G (Srivastava A K and Pandey S K Mol. and Cellular Biochem. (**182**) 135-141 (1998)).

Chen *et al*., Diabetes (**43**) 1234-1241 (1994) found that there was no difference in the mRNA abundance of PP1G between patients with Type II diabetes and control patients, suggesting that an increase in GSK-3 activity might be important in Type II diabetes. It has also recently been demonstrated that GSK-3 is overexpressed in Type II diabetic muscle and that an inverse correlation exists between skeletal muscle GSK-3α activity and insulin action (Nikoulina *et al* Glycogen Synthase Kinase-3 in Human Skeletal Muscle: Relationship To Insulin Resistance in Type II Diabetes. Diabetes **(47(1))** 0028 Page A7 (1998) (Oral presentation)). Overexpression of GSK-3β and constitutively active GSK-3β (S9A, S9E) mutants in HEK-293 cells resulted in supression of glycogen synthase activity (Eldar-Finkelman *et al*., PNAS (**93**) 10228-10233 (1996)) and overexpression of GSK-3β in CHO cells, expressing both insulin receptor and insulin receptor substrate 1 (IRS-1), resulted in an impairment of insulin action (Eldar-Finkelman and Krebs PNAS (**94**) 9660-9664 (1997)). Recent evidence for the involvement of elevated GSK-3 activity and the development of insulin resistance and type II diabetes in adipose tissue has emerged from studies undertaken in diabetes and obesity prone C57BL/6J mice (Eldar-Finkelman *et al*., Diabetes (**48**) 1662-1666 (1999)).

GSK-3 has been shown to phosphorylate other proteins *in vitro* including the eukaryotic initiation factor eIF-2B at Serine⁵⁴⁰ (Welsh *et al*., FEBS Letts (**421**) 125-130 (1998)). This phosphorylation results in an inhibition of eIF-2B activity and leads to a reduction in this key regulatory step of translation. In disease states, such as diabetes, where there is elevated GSK-3 activity this could result in a reduction of translation and potentially contribute to the pathology of the disease.

Several aspects of GSK-3 functions and regulation in addition to modulation of glycogen synthase activity indicate that inhibitors of this enzyme may be effective in treatment of disorders of the central nervous system. GSK-3 activity is subject to inhibitory phosphorylation by PI 3 kinase-mediated or Wnt-1 class-mediated signals that can be mimicked by treatment with lithium, a low mM inhibitor of GSK-3 (Stambolic V., Ruel L.and Woodgett J.R. Curr. Biol. 1996 6(12): 1664-8).

GSK-3 inhibitors may be of value as neuroprotectants in treatment of acute stroke and other neurotraumatic injuries. Roles for PI 3-kinase signalling through PKB/akt to promote neuronal cell survival are well established, and GSK-3 is one of a number of PKB/akt substrates to be identified that can contribute to the inhibition of apoptosis via this pathway (Pap & Cooper, (1998) J. Biol. Chem. **273**: 19929-19932). Evidence suggests that astrocytic glycogen can provide an alternative energy source to facilitate neuronal survival under conditions of glucose deprivation (for example see Ransom, B.R. and Fern, R. (1997) Glia **21:** 134-141 and references therein). Lithium is known to protect cerebellar granule neurons from death (D'Mello *et al*., (1994) Exp. Cell Res. **211:** 332-338 and Volonte *et al* (1994) Neurosci. Letts. **172**: 6-10) and chronic lithium treatment has demonstrable efficacy in the middle cerebral artery occlusion model of stroke in rodents (Nonaka and Chuang, (1998) Neuroreport **9**(9): 2081-2084). Wnt-induced axonal spreading and branching in neuronal culture models has been shown to correlate with GSK-3 inhibition (Lucas & Salinas, (1997) Dev. Biol. **192**: 31-44) suggesting additional value of GSK-3 inhibitors in promoting neuronal regeneration following neurotraumatic insult.

Tau and β-catenin, two known in *vivo* substrates of GSK-3, are of direct relevance in consideration of further aspects of the value of GSK-3 inhibitors in relation to treatment of chronic neurodegenerative conditions. Tau hyperphosphorylation is an early event in neurodegenerative conditions such as Alzheimer's disease (AD), and is postulated to promote microtubule disassembly. Lithium has been reported to reduce the phosphorylation of tau, enhance the binding of tau to microtubules, and promote microtubule assembly through direct and reversible inhibition of glycogen synthase kinase-3 (Hong M., Chen D.C., Klein P.S. and Lee V.M. J.Biol. Chem. 1997 272(40) 25326-32). β-catenin is phosphorylated by GSK-3 as part of a tripartite complex with axin, resulting in β-catenin being targetted for degradation (Ikeda *et al*., (1998) EMBO J. **17**: 1371-1384). Inhibition of GSK-3 activity is a key mechanism by which cytosolic levels of catenin are stabilised and hence promote β-catenin-LEF-1/TCF transcriptional activity (Eastman, Grosschedl (1999) Curr. Opin. Cell Biol. **11**: 233). Rapid onset AD mutations in presenilin-1 (PS-1) have been shown to decrease the cytosolic β-catenin pool in transgenic mice. Further evidence suggests that such a reduction in available β-catenin may increase neuronal sensitivity to amyloid mediated death through inhibition of β-catenin-LEF-1/TCF transcriptional regulation of neuroprotective genes (Zhang *et al*., (1998) Nature **395**: 698-702). A likely mechanism is suggested by the finding that mutant PS-1 protein confers decreased inactivation of GSK-3 compared with normal PS-1 (Weihl, C.C., Ghadge, G.D., Kennedy, S.G., Hay, N., Miller, R.J. and Roos, R.P.(1999) J. Neurosci. **19**: 5360-5369).

WO 97/41854 (University of Pennsylvania) discloses that an effective drug for the treatment of manic depression is lithium, but that there are serious drawbacks associated with this treatment. Whilst the precise mechanism of action of this drug for treatment of manic depression remains to be fully defined, current models suggest that inhibition of GSK-3 is a relevant target that contributes to the modulation of AP-1 DNA binding activity observed with this compound (see Manji *et al*., (1999) J. Clin. Psychiatry **60** (suppl 2): 27-39 for review).

GSK-3 inhibitors may also be of value in treatment of schizophrenia. Reduced levels of β-catenin have been reported in schizophrenic patients (Cotter D, Kerwin R, al-Sarraji S. Brion JP, Chadwich A. Lovestone S, Anderton B, Everall I. 1998 Neuroreport **9**:1379-1383 ) and defects in pre-pulse inhibition to startle response have been observed in schizophrenic patients (Swerdlow *et al*., (1994) Arch. Gen. Psychiat. **51:** 139-154). Mice lacking the adaptor protein dishevelled-1, an essential mediator of Wnt-induced inhibition of GSK-3, exhibit both a behavioural disorder and defects in pre-pulse inhibition to startle response (Lijam N, Paylor R, McDonald MP, Crawley JN, Deng CX, Herrup K, Stevens KE, Maccaferri G, McBain CJ, Sussman DJ, Wynshaw-Boris A. (1997) Cell **90**: 895-905). Together, these findings implicate deregulation of GSK-3 activity as contributing to schizophrenia. Hence, small molecule inhibitors of GSK-3 catalytic activity may be effective in treatment of this mood disorder.

The finding that transient β-catenin stabilisation may play a role in hair development (Gat *et al*., Cell (**95**) 605-614(1998)) suggests that GSK-3 inhibitors could be used in the treatment of baldness.

Certain substitued 3-amino-4-arylmaleimides are disclosed in Tetrahedron (1998), 54(9), 1745-1752; Liebigs Annalen 1894, 282, 81; BE 659639; J Amer Chem Soc 1958, 80, 1385; J. Prakt. Chem. (1979), 321(5), 787-96; Eur. J. Org. Chem. (1998), (7), 1467-1470: Chem. Heterocycl. Compd. (N. Y.) (1997), 33(1), 69-73; J. Prakt. Chem. (1987), 329(4), 587-91; Collect. Czech. Chem. Commun. (1985), 50(6), 1305-11; Tetrahedron (1984), 40(18), 3499-502; J. Prakt. Chem. (1983), 325(2), 293-300; J Prakt Chem 1983, 325 (2) 293-300; Tetrahedron (1980), 36, 1801-5; which compounds have no disclosed pharmaceutical utility.

Certain 3-amino-4-arylmaleimides are disclosed in Bioorg. Med. Chem. Lett. (1995), 5(1), 67-72; J. Med. Chem. (1992), 35(1), 177-84; Tetrahedron Lett. (1990), 31(36), 5201-4; EP 328026; Bioorg. Med. Chem. Lett. (1994), 4(24), 2845-50, which compounds are disclosed as being protein kinase C inhibitors or trypanothione reductase inhibitors. Certain 3-amino-4-arylmaleimides are disclosed in DE 4005969 and DE 4005970 as having activity as anti-allergics and immunotherapeutics.

United States Patent Number 3335147 discloses certain 3-amino-4-arylmaleimides as having topical anaesthetic activity. DE 19744257 discloses certain 3-amino-4-arylmaleimides as being tyrosine kinase inhibitors. Chem. Pharm. Bull. (1998), 46(4), 707-710 discloses certain 3-amino-4-arylmaleimides as being trypanothione reductase inhibitors. SA 672268 discloses certain 3-amino-4-arylmaleimides as being antimicrobials.

None of the above mentioned references discloses that the 3-amino-4-arylmaleimides possess GSK-3 inhibitor activity.

We have now discovered that a series of certain 3-amino-4-arylmaleimides are particularly potent and selective inhibitors of GSK-3. These compounds are indicated to be useful for the treatment and/or prophylaxis of conditions associated with a need for inhibition of GSK-3, such as diabetes, chronic neurodegenerative conditions, including dementias such as Alzheimer's disease, manic depression, mood disorders, such as schizophrenia, neurotraumatic diseases, such as acute stroke, hair loss, and cancer. Certain of these compounds are novel and such compounds comprise a further aspect of the invention. In addition, as indicated above it is considered that GSK-3 inhibitors per se are potentially useful in the treatment and/or prophylaxis of mood disorders, such as schizophrenia, neurotraumatic diseases, such as acute stroke, and for the treatment and/or prophylaxis of cancer and hair loss .

The present invention provides the compound 3-(3-chloro-4-hydroxyphenylamino)-4-(2-nitrophenyl)-1H-pyrrole-2,5-dione or a pharmaceutically acceptable salt or solvate thereof as well as pharmaceutical compositions comprising this compound as well as its use in the manufacture of a medicament for the treatment of conditions associated with a need for inhibition of glycogen synthase kinase-3 (GSK-3), such as diabetes, in particular type 2 diabetes, dementia, Alzheimer's, manic depression or cancer.

As indicated above it is considered that GSK-3 inhibitors *per se* are potentially useful in the treatment and/or prophylaxis of mood disorders, such as schizophrenia, neurotraumatic diseases, such as acute stroke, and for the treatment and/or prophylaxis of cancer and hair loss.

Accordingly, the compound is useful for the treatment and/or prophylaxis of mood disorders, such as schizophrenia, in a mammal, such as a human. which method comprises the administration of a pharmaceutically acceptable amount of a GSK-3 inhibitor.

The compound is also useful for the treatment and/or prophylaxis of neurotraumatic diseases in a mammal, such as a human.

Neurotraumatic diseases include both open or penetrating head trauma, such as caused by surgery, or a closed head trauma injury, such as caused by an injury to the head region ischaemic stroke, including acute stroke, particularly to the brain area, transient ischaemic attacks following coronary by-pass and cognitive decline following other transient ischaemic conditions.

The compound is also useful for the treatment and/or prophylaxis of cancer or hair-loss in a mammal, such as a human.

Thus, the invention also provides the use of a GSK-3 inhibitor for the manufacture of a medicament for the treatment and/or prophylaxis of mood disorders, schizophrenia, neurotraumatic diseases, cancer or hair-loss.

A suitable GSK-3 inhibitor is a compound of claim 1 or a pharmaceutically acceptable derivative thereof.

The active compounds are usually administered as the sole medicament agent but they may be administered in combination with other medicament agents as dictated by the severity and type of disease being treated. For example in the treatment of diabetes, especially Type 2 diabetes, a compound of claim 1, or a pharmaceutically acceptable derivative thereof. may be used in combination with other medicament agents. especially antidiabetic agents such as insulin secretagogues, especially sulphonylureas, insulin sensitisers. especially glitazone insulin sensitisers (for example thiazolidinediones), or with biguanides or alpha glucosidase inhibitors or the compound of claim 1, or a pharmaceutically acceptable derivative thereof, may be administered in combination with insulin.

The said combination comprises co-administration of a compound of claim 1, or a pharmaceutically acceptable derivative thereof, and an additional medicament agent or the sequential administration of a compound of claim 1, or a pharmaceutically acceptable derivative thereof, and the additional medicament agent.

Co-administration includes administration of a pharmaceutical composition which contains both a compound of claim 1, or a pharmaceutically acceptable derivative thereof. and the additional medicament agent or the essentially simultaneous administration of separate pharmaceutical compositions of a compound of claim 1, or a pharmaceutically acceptable derivative thereof, and the additional medicament agent.

The compositions of the invention are preferably adapted for oral administration. However, they may be adapted for other modes of administration.

The compositions may be in the form of tablets, capsules, powders, granules. lozenges, suppositories, reconstitutable powders, or liquid preparations, such as oral or sterile parenteral solutions or suspensions.

In order to obtain consistency of administration it is preferred that a composition of the invention is in the form of a unit dose.

Preferably the composition are in unit dosage form. A unit dose will generally contain from 0.1 to 1000 mg of the active compound.

Generally an effective administered amount of a compound of the invention will depend on the relative efficacy of the compound chosen, the severity of the disorder being treated and the weight of the sufferer. However, active compounds will typically be administered once or more times a day for example 2, 3 or 4 times daily, with typical total daily doses in the range of from 0.1 to 800 mg/kg/day.

Suitable dose forms for oral administration may be tablets and capsules and may contain conventional excipients such as binding agents, for example syrup, acacia, gelatin. sorbitol, tragacanth, or polyvinylpyrrolidone; fillers, for example lactose. sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending. filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example. emulsions. syrups, or elixirs. or may be presented as a dry product for reconstitution with water or other suitable vehicle before use. Such liquid preparations may contain conventional additives such as suspending agents, for example sorbitol, syrup. methyl cellulose, gelatin. hydroxyethylcellulose, carboxymethylcellulose, aluminium stearate gel, hydrogenated edible fats; emulsifying agents, for example lecithin, sorbitan monooleate. or acacia; non-aqueous vehicles (which may include edible oils), for example almond oil, fractionated coconut oil, oily esters such as esters of glycerine, propylene glycol, or ethyl alcohol: preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic. a preservative and buffering agents can be dissolved in the vehicle. To enhance the stability. the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The formulations mentioned herein are carried out using standard methods such as those described or referred to in reference texts such as the British and US Pharmacopoeias, Remington's Pharmaceutical Sciences (Mack Publishing Co.), Martindale The Extra Pharmacopoeia (London, The Pharmaceutical Press) or the above mentioned publications.

Suitable methods for preparing and suitable unit dosages for the additional medicament agent, such as the antidiabetic agent mentioned herein include those methods and dosages described or referred to in the above mentioned reference texts.

### GSK-3 Assays

Types of GSK-3 assay used to test the compound of the invention include the following:
**Type 1**: The GSK-3 specific peptide used in this assay was derived from the phosphorylation site of glycogen synthase and its sequence is:
   YRRAAVPPSPSLSRHSSPHQ(S)EDEEE. (S) is pre-phosphorylated as is glycogen synthase in vivo and the three consensus sites for GSK-3 specific phosphorylation are underlined. The buffer used to make up the glycogen synthase peptide and [γ-³³P] ATP consisted of MOPS 25mM. EDTA 0.2mM. MgAcetate 10mM, Tween-20 0.01% and mercaptoethanol 7.5mM at pH 7.00.
   The compounds were dissolved in dimethyl sulphoxide (DMSO) to a final concentration of 100mM. Various concentrations were made up in DMSO and mixed with the substrate (GSK-3 peptide) solution (to a final concentration 20uM) described in the above section along with rabbit or human GSK-3α and GSK-3β (final concentration 0.5U/ml enzyme). The reactions were initiated with the addition of [γ-³³P] ATP (500cpm/pmole) spiked into a mixture of ATP (final concentration of 10µM). After 30 min at room temperature the reaction was terminated by the addition of 10µl of H₃PO₄ / 0.01% Tween-20 (2.5%). A volume (10µl) of the mixture was spotted onto P-30 phosphocellulose paper (Wallac & Berthold, EG&G Instruments Ltd, Milton Keynes). The paper was washed four times in H₃PO₄ (0.5%), 2 mins for each wash, air dried and the radioactive phosphate incorporated into the synthetic glycogen synthase peptide, which binds to the P-30 phosphocellulose paper. was counted in a Wallac microbeta scintillation counter.
   Analysis of Data: Values for IC₅₀ for each inhibitor were calculated by fitting a four-parameter logistic curve to the model : cpm=lower+(upper-lower) /(1 + (concentration/ IC₅₀) ^{slope}).
Type 2: This protocol is based on the ability of the kinase to phosphorylate a biotinylated 26 mer peptide, sequence of which derived from the phosphorylation site of glycogen synthase and its sequence is Biot- YRRAAVPPSPSLSRHSSPHQ(S)EDEEE, with (S) is a pre-phosphorylated serine as is glycogen synthase in vivo and the three consensus sites for GSK-3 specific phosphorylation are underlined. The phosphorylated biotinylated peptide is then captured onto streptavidin coated SPA beads (Amersham Technology), where the signal from the 33P is amplified via the scintillant contained in the beads.
   The kinase was assayed at a concentration of 10 nM final in 25 mM MOPS buffer, pH 7.0 containing 0.0 1 % Tween-20, 7.5 mM 2-mercaptoethanol, 10 mM Magnesium acetate. and 10 uM [γ-³³P]-ATP. After 60 minutes incubation at room temperature, the reaction was stopped by addition of 50 mM EDTA solution containing the Streptavidin coated SPA beads to give a final 0.5 mgs of beads per assay well in a 384 microtiter plate format.
   10 mM stock solutions of the compounds of the invention in 100% DMSO are generated as a first step in the screening process. The second step involves the creation of dose response plates where these compounds are diluted across the plate where the final low and high concentrations are to be 0.008 and 10 uM final in the kinase assay. The third step involves the creation of the assay plates. This is achieved by transferring the compounds from four 96 dose response plates to one 384 assay plate on the Robocon Robolab system. The fourth step is to perform the assay as described and count the resulting plates in the Trilux (Wallac 1450 microbeta liquid scintillation and luminescence counter). The final step is data acquisition and analysis where IC₅₀ values are generated for each compound in duplicate by fitting a four parameter logistic curve to the model : cpm = lower + (upper-lower) / (1 + (concentration / IC₅₀) ^{slope}) in a batch manner.

The most potent compounds of the present invention show IC₅₀ values in the range of from between 10 to 100 nM.

No adverse toxicological effects are expected for the compound of the invention, when administered in accordance with the invention.

### Compound 1

### 3-(3-Bromophenylamino)-4-(4-chlorophenyl)-1H-pyrrole-2,5-dione

A solution of 3-bromoaniline (2.27 mL, 0.020 mol) and 3-chloro-4-(4-chlorophenyl)-1H-pyrrole-2.5-dione (2.02 g, 0.0083 mol; prepared by analogy with the methods described in WO97/34890 and Wiley, R.H. and Slaymaker, S.C. J. Am. Chem. Soc. (80) 1385 (1958)) in methanol (50 mL) was heated at reflux for 40 hours, cooled and concentrated. The residue was acidified with aqueous hydrochloric acid (1M, 200 mL) and extracted with ethyl acetate (3 x 200 mL). The combined organic solutions were washed with water and brine, dried with magnesium sulphate, evaporated and the residue chromatographed on silica gel using dichloromethane-diethyl ether (gradient from 100:0 to 95:5 v/v) as eluent to afford the title compound as a solid.
¹H NMR (DMSO-d₆): δ6.70-7.30 (8H, m), δ9.65 (1H, br), δ10.90 (1H, br).
MS (APCI +ve): [M+H]⁺ at m/z 377/379/381 (C₁₆H₁₀BrClN₂O₂ requires [M+H]⁺ at m/z 377/379/381).

### Compound 2

### 3-(4-Benzoylphenylamino)-4-(4-chlorophenyl)-1H-pyrrole-2,5-dione

A sealed tube (comprising threaded glass tube with resealable cap) containing a mixture of 4-aminobenzophenone (0.147 g, 0.75 mmol), 3-chloro-4-(4-chlorophenyl)-1H-pyrrole-2,5-dione (0.061 g, 0.25 mmol) and 1-methyl-2-pyrrolidinone (0.5 mL) was irradiated in a microwave reactor for 12 minutes at 100 Watts. The mixture was diluted with aqueous hydrochloric acid (5 mL) and extracted with ethyl acetate (2 x 5 mL). The combined organic solutions were evaporated and the residue chromatographed on silica gel using dichloromethane as eluent to afford the title compound as a solid.
¹H NMR (DMSO-d₆): δ6.85 (2H, d), δ7.00 (2H, d), δ7.25 (2H, d), δ7.35 (2H, d). δ7.50-7.70 (5H. m), δ9.95 (1H, s), δ10.95 (1H, s)
MS (APCI -ve): [M]-. at m/z 402/404 (C₂₃H₁₅ClN₂O₃ requires [M]⁻. at m/z 402/404)

### Compound 3

### 3-(3-Bromo-4-methylphenylamino)-4-(3-nitrophenyl)-1H-pyrrole-2,5-dione

A mixture of 3-bromo-4-methylaniline (0.220 g, 1.18 mmol), 3-chloro-4-(3-nitrophenyl)-1H-pyrrole-2,5-dione (0.100 g, 0.40 mmol) and 1-methyl-2-pyrrolidinone (1.0 mL) was heated in an oil bath at 200°C for 51 minutes. The mixture was diluted with aqueous hydrochloric acid (5 mL) and extracted with ethyl acetate (5 mL). The combined organic solutions were evaporated and the residue chromatographed on silica gel using dichloromethane as eluent to afford the title compound, a solid, following trituration with dichloromethane-hexane (90:10 v/v).
¹H NMR (CDCl₃): δ2.24 (3H, s), δ6.65-7.70 (7H, m, reduces to 5H on D₂O exchange) and 88.05 (2H. m).
MS (APCI -ve): [M-H]⁻ at m/z 400/402 (C₁₇H₁₂BrN₃O₄ requires [M-H]- at m/z 400/402).

### Compound 4

### 3-(4-Methylphenylamino)-4-(4-hydroxyphenyl)-1H-pyrrole-2,5-dione

A mixture of 3-hydroxy-4-(4-hydroxyphenyl)-1H-pyrrole-2,5-dione (103 mg, 0.5 mmol) and 4-methylaniline (59 mg, 0.55 mmol) in 1-methyl-2-pyrrolidinone (1mL) was heated in a sealed tube at 150°C for 24hours. The reaction mixture was dissolved in ethyl acetate(20 mL) and washed with 1N HCl (2 x 20 mL), water (3 x 20 mL) and brine (20 mL). The solution was dried over magnesium sulphate, evaporated and the residue chromatographed on silica gel using dichloromethane-diethyl ether (gradient from 100:0 to 90:10 v/v) as eluent to afford the title compound as a solid.
¹H NMR (DMSO-d₆): δ2.35 (3H, s), δ6.50 (2H, d), δ6.64 (2H, d), δ6.77 (2H, d), δ6.90 (2H, d), δ9.26 (1H, br), δ9.44 (1H, br), δ10.64 (1H, br).
MS (APCI +ve): [M+H]⁺ at m/z 295 (C ₁₇H₁₄N₂O₃ requires [M+H]⁺ at m/z 295).

### Compound 5

### 3-(N-Methyl-N-phenylamino)-4-(indol-3-yl)-1H-pyrrole-2,5-dione.

A mixture of 3-(N-methyl-N-phenylamino)-4-(indol-3-yl)-1-methyl-1H-pyrrole-2,5-dione (Table B, Example B1; 2.00 g, 0.006 mol), aqueous potassium hydroxide solution (10% w/v, 2 L), ethanol (50 mL) and *n*-butanol (200 mL) was heated at reflux for 5 hours. The cooled reaction mixture was filtered and the filtrate acidified to pH 1 by addition of conc. hydrochloric acid. The mixture was cooled to 0°C and the resulting solid filtered. washed with water and recrystallised from acetonitrile to give the corresponding maleic anhydride. This anhydride (0.4 g, 1.25 mmol) was suspended in a mixture of concentrated aqueous ammonium hydroxide and DMF and heated in stainless steel bomb at 130°C for 4 hours. The resulting mixture was diluted with water and extracted with dichloromethane and the dried organic solution evaporated to give a solid. This was chromatographed on silica gel using a gradient of 0-5% (v/v) of methanol in dichloromethane as eluent to afford the title compound. a solid.
¹H NMR (DMSO-d₆): δ3.07 (3H, s), δ6.75-7.45 (9H, m), δ7.68 (1H, s), δ10.70 (1H, br) and δ11.70 (1H, br).
MS (APCI +ve): [M+H]⁺ at m/z 318 (C ₁₉H₁₅N₃O₂ requires [M+H]⁺ at m/z 318).

Further elution of the chromatography column afforded 3-amino-4-(indol-3-yl)-1H-pyrrole-2.5-dione (Table B, Example B2) as a byproduct.

### Compound 6

### 3-(Indan-5-ylamino)-4-(3-aminophenyl)-1H-pyrrole-2,5-dione

3-(Indan-5-ylamino)-4-(3-nitrophenyl)-1H-pyrrole-2,5-dione (Table A, Example A359; 0.3 g, 0.9 mmol) and 10% Pd/C (60 mg) in ethanol (25 mL) was hydrogenated at atmospheric temperature and pressure for 2 hours. The reaction mixture was filtered through Kieselguhr and the filtrate concentrated in vacuo to give an orange solid. The crude product was taken up in dichloromethane (10 mL) and treated with di-tert-butyl dicarbonate (0.216 g, 1 mmol) and the mixture stirred at ambient temperature for 18 hours. The reaction mixture was poured into saturated aqueous sodium bicarbonate (10 mL) and extracted into dichloromethane (3x10 mL). The combined organics were washed with brine, dried over anhydrous sodium sulfate and concentrated in vacuo. Chromatography on silica gel using dichloromethane-methanol gave the product *amine* as an orange powder.
¹H NMR (DMSO-d₆): δ1.85 (2H, quintet), δ2.50 (2H, t), δ2.66 (2H, t), δ4.82 (2H, s), δ5.89 (1H, d), δ6.36 (2H, m), δ6.47 (1H, s), δ6.25 (2H, m), δ6.85 (1H, d), δ9.13 (1H, br) and δ10.58 (1H, br).
MS (APCI +ve): [M+H]⁺ at m/z 320 (C₁₉H₁₇N₃O₂ requires [M+H]⁺ at m/z 320)

### Compound 7

### 3-(Indan-5-ylamino)-4-(3-acetylaminophenyl)-1H-pyrrole-2,5-dione

3-(Indan-5-ylamino)-4-(3-nitrophenyl)-1H-pyrrole-2,5-dione (Table A, Example A359; 0.3 g, 0.9 mmol) and 10% Pd/C (60 mg) in ethanol (25 mL) was hydrogenated at atmospheric temperature and pressure for 2 hours. The reaction mixture was filtered through Kieselguhr and the filtrate concentrated in vacuo to give an orange solid. The crude product was taken up in dichloromethane (5 mL) and treated with acetic anhydride (85 µL, 0.9 mmol) and stirred for 3 hours at ambient temperature. The reaction mixture was poured onto saturated aqueous sodium bicarbonate solution (10 mL) and extracted into ethyl acetate (3x10 mL). The combined organics were washed with brine, dried over anhydrous sodium sulfate and concentrated in vacuo. Chromatography on silica gel using dichloromethane-methanol gave the desired compound as an orange powder.
¹H NMR (DMSO-d₆): δ1.83(2H, quintet), δ2.02 (3H, s), δ2.45 (2H, t). δ2.66 (2H, t), δ6.41 (2H, m), δ6.59 (1H, d), δ6.84 (2H, d), δ6.90 (1H, t), δ7.38 (1H, d), δ9.30 (1H, bs), δ9.68 (1H, s) and δ10.61 (1H, bs)]
MS (APCI -ve): [M-H]⁻ at m/z 360 (C₂₁H₁₉N₃O₃ requires [M-H]- at m/z 360).

### Compound 8

### 3-(Indan-5-ylamino)-4-[3-[(3-fluorophenylaminocarbonyl)amino]phenyl]-1H-pyrrole-2,5-dione

3-(Indan-5-ylamino)-4-(3-aminophenyl)-1H-pyrrole-2,5-dione (Table A, Example A599; 0.08 g, 0.3 mmol) in dichloromethane (10 mL) was was treated with 3-fluorophenyl isocyanate (0.038mg, 0.3 mmol). The mixture was shaken on an orbital shaker for 72 hours. Saturated aqueous sodium bicarbonate (5 mL) was added, shaking continued for 5 minutes and the organic layer transferred directly onto a column of silica gel. Elution with dichloromethane gave the product as a yellow solid.
¹H NMR (DMSO-d₆): δ1.78 (2H, quintet), δ2.44 (2H, t), δ2.62 (2H, t), δ6.47 (2H, m), δ6.61 (1H, dd), δ6.83 (2H, m), δ6.93 (2H, m), δ7.09 (1H, dd), δ7.28 (2H, m), δ7.45 (1H, dd), δ8.42 (1H, br), δ8.72 (1H, br), δ9.30 (1H, br) and δ10.65 (1H, br).
MS (APCI -ve) [M]⁻ at m/z 456 (C₂₆H₂₁FN₄O₃ requires [M]⁻ at m/z 456).

### Compound 9

### 3-(Indan-5-ylamino)-4-[3-(benzoylamino)phenyl]-1H-pyrrole-2,5-dione

3-(5-Indan-5-ylamino)-4-(3-aminophenyl)-1H-pyrrole-2,5-dione (Table A, Example A599; 0.100 g, 0.3 mmol) in dichloromethane (3 mL) was added to a solution of benzoic acid (0.042 g, 0:33 mmol), 1-hydroxybenzotriazole (0.047 g, 0.33 mmol) and 1-(3-dimethylaminopropyl)-3-ethylcarbodiimide hydrochloride (0.063 g, 0.33 mmol in dichloromethane (5 mL). The mixture was shaken on an orbital shaker for 72 hours. Saturated aqueous sodium bicarbonate (5 mL) was added. shaking continued for 5 minutes and the organic layer transferred directly onto a column of silica gel. Elution with dichloromethane gave the product as a yellow solid.
¹H NMR (DMSO-d₆): δ1.83 (2H, quintet), δ2.43 (2H, t), δ2.57 (2H, t), δ6.42 (1 H, s), δ6.30 (2H, m), δ6.83 (1H, d), δ7.02 (1H, t), δ7.22 (1H, s), δ7.56 (4H, m), δ7.86 (2H, dd), δ9.38 (1H, br), δ9.98 (1H, br) and δ10.68 (1H, bs).
MS (APCI -ve): [M-H]- at m/z 422 (C₂₆H₂₁N₃O₃ requires [M-H]- at m/z 422)

### Compound 10

### 3-[4-(2-Aminoethyl)phenylamino]-4-(2-methoxyphenyl)-1H-pyrrole-2,5-dione

A solution of 3-[4-[2-(*t*-butoxycarbonylamino)ethyl]phenylamino]-4-(2-methoxyphenyl)-1H-pyrrole-2.5-dione (0.060 g. 0. 13 mmol) and trifluoroacetic acid (4 drops) in dry DCM (5 mL) was stirred for 18 hours at room temperature. The suspension was diluted with ethyl acetate (10 mL), poured onto sodium bicarbonate (20 mL) and extracted with ethyl acetate (3 x 10 mL). The combined organic solutions were washed with brine. dried with magnesium sulfate. evaporated and the residue triturated with a mixture of hexane-dichloromethane (95:5 v/v) to afford the title compound as an orange solid.
¹H NMR (CDCl₃); δ1.52 (2H, br), δ2.59 (2H, t), δ2.83 (2H, t), δ3.16 (3H, s), δ6.44 ( 1 H, d), δ6.58 (2H. d), δ6.79 (2H,d), δ6.97-6.93 (1H, m), δ7.22-7.17 (3H, m) and δ7.33 (1H, d).
MS (APCI +ve): [M+H]⁺ at m/z 338 (C₁₉H₁₉N₃O₃ requires [M+H]⁺ at 338).

### Compound 11

### 3-(3-Fluoro-4-methylphenylamino)-4-[4-(methoxycarbonyl)phenyl]-1H-pyrrole-2,5-dione

A mixture of 3-(3-Fluoro-4-methylphenyl-amino)-4-(4-iodophenyl)-1H-pyrrole-2,5-dione (Example A705, 126 mg, 0.3 mmol), tetrakis(triphenyl phosphine)-palladium(0) (35 mg, 0.03 mmol) and methanol (10 mL) was placed in a 50mL two necked round bottomed flask. One arm of the flask was sealed with a septum and to the other arm was fitted a reflux condenser, topped with a multiway tap connected respectively to vacuum. a carbon monoxide cylinder and to a balloon. Using the multiway tap, the flask was alternately evacuated and flushed with carbon monoxide, and the process repeated several times to unsure an atmosphere of carbon monoxide within the flask. The balloon was charged with carbon monoxide and this was then opened to the reaction flask for the duration of the reaction in order to maintain a slight positive pressure of carbon monoxide within the flask. Triethylamine (100 uL, 0.7 mmol) was added and the mixture heated at reflux for 16 hours. The mixture was cooled and diluted with ethyl acetate and the resulting solution washed with aqueous hydrochloric acid (1M, 50 mL), water (50 mL) and brine (50 mL). The organic solution was dried over magnesium sulphate and evaporated to afford a solid. This was chromatographed on silica gel using dichloromethane-ether (98:2 v/v) as eluent to afford the title compound, a solid.
¹H NMR (CDCl₃); δ2.14 (3H, s), δ3.90 (3H, s), δ6.35-7.30 (7H, m) and δ7.82 (2H, m). MS (APCI +ve): [M+H]⁺ at m/z 355 (C₁₉H₁₅FN₂O₄ requires [M+H]⁺ at 355).

### Compound 12

### 3-[4-[2-[N-[6-(Acetylamino)hexyl]aminocarbonyl]ethyl]phenylamino]-4-(3-nitrophenyl)-1H-pyrrole-2,5-dione

A solution of triethylamine (81 mg, 0.8 mmol) in dry N, N-dimethylformamide (5 mL) was added to a mixture of 3-[4-[2-(hydroxycarbonyl)ethyl]phenylamino]-4-(3-nitrophenyl)-1H-pyrrole-2,5-dione (Example A763, 152 mg, 0.4 mmol), N-(6-aminohexyl)acetamide hydrochloride (78 mg, 0.4 mmol), 1-(3-dimethylamino-propyl)-3-ethylcarbodiimide hydrochloride (77 mg, 0.4 mmol) and 1-hydroxybenzotriazole (54 mg, 0.4 mmol) and the resulting mixture stirred at room temperature for 18 hours. The mixture was diluted with ethyl acetate (25 mL) and washed successively with water (2 x 25 mL), saturated aqueous sodium bicarbonate solution (25 mL), water (2 x 25 mL), brine (25 mL), dried over magnesium sulphate and concentrated. The residue was redissolved in dichloromethane-methanol (1:1 v/v), filtered and evaporated to afford the title compound as a foam.
¹H NMR (DMSO-d₆); δ1.10-1.40 (8H, m), δ1.77 (3H, s), δ2.15 (2H, m), δ2.55 (2H, m), δ3.00 (4H, m), δ6.62 (2H, d), δ6.77 (2H, d), δ7.20-7.90 (6H, m), δ9.80 (1H, br) and δ10.85 (1H, br).
MS (APCI +ve): [M+H]⁺ at m/z 522 (C₂₇H₃₁N₅O₆ requires [M+H]⁺ at 522).

### Compound 13

### 3-[4-(trans-2-carboxyethenyl)phenylamino]-4-(4-chlorophenyl)-1H-pyrrole-2,5-dione

A mixture of *trans*-4-aminocinnamic acid (0.205 g, 1.26 mmol), 3-chloro-4-(4-chloroghenyl)-1H-pyrrole-2,5-dione (0.123 g, 0.51 mmol) and 1-methyl-2-pyrrolidinone (1.0 mL) was heated in a sealed tube in a hotblock set at 69°C for 28.5 hours. The mixture was diluted with aqueous hydrochloric acid (10 mL) and extracted with ethyl acetate (2x20 mL). The combined organics were washed with brine (2x10 mL), dried over anhydrous magnesium sulphate and evaporated to dryness. The residue was triturated with a mixture of dichloromethane and ethyl acetate to afford the title compound as a solid.
¹H NMR (DMSO-d₆): δ6.35 (1H, d), 6.74 (2H, d), 6.99 (2H, d), 7.19(2H, d), 7.35 (2H, d), 7.42 (1H, d), 9.76 (1H, br), 10.89(1H, br) and δ12.23 (1H, br).
MS (APCI +ve): [M+H]⁺ at m/z 369/371 (C₁₉H₁₃N₂O₄ requires [M+H]⁺ at m/z 369/371).

### Compound 14

### 3-[4-(trans-2-carbamoylethenyl)phenylamino]-4-(4-chlorophenyl)-1H-pyrrole-2,5-dione

3-[4-[*trans*-2-(ethoxycarbonyl)ethenyl]phenylamino)-4-(4-chlorophenyl)-1H-pyrrole-2,5-dione (50mg, 0.126mmol) was dissolved in 2N methanolic ammonia (5ml) and allowed to stand at room temp for 12days. Aqueous ammonia (d 0.88, 5ml) was added and the solution stood at room temp for a further 8 days. The mixture was evaporated to dryness and the residue triturated with methanol then ether to give the title compound as a solid.
1H NMR (DMSO-d₆): δ10.75(1H, br), δ9.7 (1H, br), δ7.44 (1H, br), δ7.2 (5H, m), δ7.2 (3H, m), δ6.74 (2H, d), δ6.41 (1H, d).
MS (APCI +ve): [M+H]⁺ at m/z 368/370 (C₁₉H₁₄ClN₃O₃ requires [M+H]⁺ at m/z 368/370).

### Compound 15

### 3-(Indol-1-yl)-4-(3-nitrophenyl)-1H-pyrrole-2,5-dione

Sodium hydride (60% dispersion in mineral oil, 30 mg, 0.75 mmol) was added to a solution of indole (88 mg, 0.75 mmol) in THF (2 mL) at room temperature. The mixture was stirred for 30 minutes prior to the addition of a solution of 1-(*tert*-butyldimethylsilyl)-3-chloro-4-(3-nitrophenyl)-1H-pyrrole-2,5-dione (Procedure method 1, 180 mg, 0.5 mmol) in THF (1 mL). The mixture was stirred for 45 minutes then diluted with ethyl acetate (80 mL), washed with dilute hydrochloric acid (20 mL), dried (MgSO₄) and concentrated. The residue was chromatographed on silica gel using a gradient of hexane-ethyl acetate to afford the title compound, a solid.
¹H NMR (CD₃OD); δ6.42 (1H, d), 6.77 (1H, d), 6.82 (1H, t), 7.00-7.60 (5H, m) and 8.05-8.25 (2H, m).
MS (APCI +ve): [M+H] ⁺ at m/z 334 (C₁₈H₁₁N₃O₄ requires [M+H]⁺ at 334).

### Compound 16

### 3-Amino-4-(3-nitrophenyl)-1H-pyrrole-2,5-dione

3-chloro-4-(3-nitrophenyl)-1H-pyrrole-2,5-dione (1.0 g, 4 mmol) was suspended in a mixture of ethanol (20 mL) and aqueous 880 ammonia (5 mL) and the mixture heated to 80°C whilst ammonia gas was bubbled through the mixture for 4 hours. The mixture was cooled and concentrated and the residue chromatographed on silica gel using hexane-ethyl acetate (gradient from 1:1 v/v) as eluent to afford the title compound as a solid.
¹H NMR (CD₃COCD₃); δ6.77 (2H, br), 7.60 (1H, t), 8.04 (2H, m), 8.50 (1H, t) and 9.33 (1H, br).
MS (APCI +ve): [M+H] ⁺ at m/z 234 (C₁₀H₇N₃O₄ requires [M+H]⁺ at 234).

### Compound 17

### 3-[4-[2-methoxyethylaminocarbonylmethylthio]phenylamino]-4-(4-chlorophenyl)-1H-pyrrole-2,5-dione

A solution of 2-methoxyethylamine in THF (0.32M, 1 mL) was added to a mixture of 3-[4-(carboxymethylthio)phenylamino]-4-(4-chlorophenyl)-1H-pyrrole-2,5-dione (Example A941, 117 mg, 0.3 mmol), 1-(3-dimethylamino-propyl)-3-ethylcarbodiimide hydrochloride (57 mg, 0.3 mmol) and 1-hydroxybenzotriazole (40 mg, 0.3 mmol) in dry THF (1 mL). The resulting solution was stirred at room temperature for 57 hours, then diluted with ethyl acetate (50 mL) and washed with dilute hydrochloric acid (1M, 50 ml), water (50 mL) and brine (50 mL), dried over magnesium sulphate and evaporated. The resulting gum was chromatographed on silica gel using dichloromethane-methanol (98:2 v/v) as eluent to afford the title compound, a solid.
¹H NMR (DMSO-d₆) d 3.20 (3H, s), 3.21 (2H, m), 3.25 (2H, t), 3.50 (2H, s), 6.60-7.20 (8H, m), 8.10 (1H, t, exchanges with D₂O), 9.65 (1H, br, exchanges with D₂O) and 10.82 (1H, br, exchanges with D₂O).
MS (APCI+ve) [M+H]⁺ at m/z 446/448. C₂₁H₂₀ClN₃O₄S requires [M+H]⁺ at m/z 446/448.

### Compound 18

### 3-(2-Methoxyethylamino)-4-(4-iodophenyl)-1H-pyrrole-2,5-dione

A solution of 3-(3-fluoro-4-methylphenylamino)-4-(4-iodophenyl)-1H-pyrrole-2,5-dione (Example A705, 126 mg, 0.3 mmol) and 2-methoxyethylamine (0.2 mL, 2.3 mmol) in DMF (2 mL) was stirred at room temperature for 113 hours then diluted with hydrochloric acid (0.5M, 50 mL) and extracted with ethyl acetate (50 mL). The ethyl acetate solution was washed with water (2 x 50 mL) and brine (50 mL), dried over magnesium sulphate and evaporated. The residue was chromatographed on silica gel using dichloromethane-diethyl ether (99:1 v/v) as eluent to afford the title compound, a solid.
¹H NMR (CDCl₃): 3.25 (2H, m), 3.35 (3H, s), 3.40 (2H, t), 5.67 (1H, br, exchanges with D₂O), 6.95 (1H, br, exchanges with D₂O), 7.05 (2H, d) and 7.70 (2H, d). MS (APCI+ve) [M+]⁺ at m/z 373. C₁₃H₁₃IN₂O₃ requires [M+H]⁺ at m/z 373.

### Compound 19

### 3-Amino-1-[4-(4-chlorophenyl)-2,5-dioxo-1H-pyrrol-3-yl]pyridinium chloride

A mixture of 3-chloro-4-(4-chlorophenyl)-1H-pyrrole-2,5-dione (100 mg, 0.41 mmol) and 3-aminopyridine (42.7 mg, 0.45 mmol) in dry THF (2.5 mL) was heated at 50°C for 2 hours then stirred at room temperature overnight. The resulting suspension was filtered and the solid washed with dichloromethane (20 mL), then hexane (10 mL) to give the title compound as a solid.
¹H NMR (DMSO): δ7.07 (2H,br), δ7.43 (2H,d), δ7.61 (2H,d), δ7.93-7.81 (2H,m), δ8.10-8.07 (2H,m) and δ12.07 (1H,br).
MS (APCI+ve): [M+H]⁺ at m/z 301/303 (C₁₅H₁₁N₃O₂Cl requires [M+H]⁺ at m/z 301/303)

### Compound 20

### 3-[5-methoxy-6-[4-ethylpiperazin-1-yl]-indolin-1-yl]-4-[3-fluorophenyl]-1H-pyrrole-2,5-dione

A solution of 3-chloro-4-(3-fluorophenyl)-1H-pyrrole-2,5-dione (100 mg, 0.44 mmol.), 5-methoxy-6-[4-ethylpiperazin-1-yl]-indoline (156 mg, 0.44 mmol.) and triethylamine (0.12 mL, 0.88 mmol.) in dry 1-methylpyrrolidin-2-one (2 mL) was heated under argon at 65 C for 36 h. The mixture was allowed to stand overnight at RT then diluted with water (80 mL) and extracted with ethyl acetate (3 x 60 mL). The combined organic solutions were washed with water (2 x 60 mL), brine, dried with magnesium sulphate, evaporated and the residue triturated with a mixture of dichloromethane and hexane to afford the title compound as a solid.
¹H NMR (DMSO-d₆): δ10.80 (1H, br), δ 7.23-7.17 (1H, m), δ 7.00 (1H, t), δ 6.92-6.85 (3H, m), δ 5.44 (1H, s), δ 4.42 (2H, t), δ 3.71 (3H, s), δ 3.12 (2H, t), δ 2.29 (10H, br.s), δ 0.96 (3H, t)
MS (APCI+ve) : [M+H]⁺ at m/z 451 (C₂₅H₂₇N₄O₃F requires [M+H]⁺ at m/z 451)

### Compound 21

### 3-[2-(Hydroxymethyl)indolin-1-yl]-4-(3-nitrophenyl)-1H-pyrrole-2,5-dione single enantiomer

A solution of racemic 3-[2-(Hydroxymethyl)indolin-1-yl]-4-(3-nitrophenyl)-1H-pyrrole-2.5-dione (Example D102, 30mg) in acetone (1ml) was separated into it's two enantiomers by repeated high pressure liquid chromatography of aliquots of the solution. The chromatography was performed on a waters 6000 instrument equipped with a 10mm chiracel AD column using hexane-ethanol (85:15 v/v) as eluent at 5 ml min⁻¹. The solvent was removed at reduced pressure to give the separated enantiomers as solids. Enantiomer 1 (12mg, 100% chiral purity), enantiomer 2 ( 11mg, 96% chiral purity).
¹H NMR (MeOH): δ 2.07-2.25 (2H,m), 2.48 (1H,dd), 2.65 (1H,dd), 4.10 (1H,hept), 4.45 (1H,d), 5.33 (1H,t), 5.52 (1H,t), 5.95 (1H, d), 6.16 (1H,t), 6.42 (1H, d), 6.78 (1H,dd), 6.85 (1H, d).
MS (APCI+ve) [M⁺H]⁺ at m/z 366. (C₁₉H₁₅IN₃O₅ requires [M+H]⁺ at m/z 366).

### Compound 22

### 3-(3,5-Di-fluorophenylamino)-4-(2,3-di-fluorophenyl)-1H-pyrrole-2,5-dione

A solution of 3,5-difluoroaniline (161 mg, 0.00125 mol) and 3-chloro-4-(2,3-difluorophenyl)-1H-pyrrole-2,5-dione (122 mg, 0.0005mol) in methanol (2 mL) was heated in a sealed tube at 65°C for 8 days. The mixture was acidified with aqueous hydrochloric acid ( I M) and extracted with ethyl acetate. The combined organic solutions were washed with water and brine. dried with magnesium sulphate, evaporated and the residue triturated with hexane-dichloromethane (95:5 v/v) to afford the title compound as a solid.
¹H NMR (DMSO-d₆): δ6.40 (2H, m), δ6.75 (1H, m), δ7.00-7.40 (3H, m), δ10.00 (1H, br) and δ11.00 (1H, br).
MS (APCI +ve): [M+H]⁺ at m/z 337 (C₁₆H₈F₄N₂O₂ requires [M+H]⁺ at m/z 337).

### Procedure Method 1

### 1-(tert-Butyldimethylsilyl)-3-chloro-4-(3-nitrophenyl)-1H-pyrrole-2,5-dione

Triethylamine (1.1 mL, 8 mmol) was added to a stirred suspension of *tert*-butylchlorodimethylsilane (0.66 g, 4.4 mmol) and 3-chloro-4-(3-nitrophenyl)-1H-pyrrole-2,5-dione (1.0 g, 4 mmol) in dichloromethane (15 mL) at room temperature. The mixture was stirred overnight then chromatographed directly on silica gel using a hexane-acetone gradient to afford the title compound.
¹H NMR (CDCl₃); δ0.51 (6H, s), 0.98 (9H, s), 7.70 (1H, t), 8.27 (2H, m) and 8.80 (1H, m).
MS (APCI -ve): [M-H] - at m/z 366/368 (C₁₆H₁₉ClN₂O₄Si requires [M-H]⁻ at 366/368).

The following additional procedures (Procedure Methods 2 & 3) serve to illustrate a typical preparation of a non commercial aniline, by a method analogous to that described in *Synthesis* 1994, 1413.:-

### Procedure Method 2

### 3-[(4-Nitrophenyl)thio]benzoic acid

A suspension of potassium carbonate (18g) in acetone (140 mL) at ambient temperature was treated with 3-mercaptobenzoic acid (10g, 64.4 mmol, I eq) followed by 4-nitrofluorobenzene (18g, 127.7 mmol, 2 eq). The resultant mixture was stirred for 18h and then poured onto saturated sodium bicarbonate and washed with ethyl acetate. The basic aqueous layer was acidified with 5N HCl and extracted into ethyl acetate (3x100 mL). The combined organics were dried with anhydrous sodium sulphate and concentrated *in vacuo* to give the product as a solid.
¹H NMR (DMSO): δ7.35 (2H, d), 7.66 (1H, t), 7.81 (1H, m), 8.06 (2H, m), 8.16 (2H, d), and 13.31 (1H, bs).
MS (APCI-ve): [M-H]⁻ at m/z 274 (C₁₃H₉NO₄S requires [M-H]- at m/z 274)

### Procedure Method 3

### 3-[(4-Aminophenyl)thio]benzoic acid

A mixture of 3-[(4-nitrophenyl)thio]benzoic acid (11.2g, 40.7 mmol) and 10% Pd/C (0.5g) in ethanol (250 mL) was hydrogenated at atmospheric temperature and pressure for 24h. The mixture was filtered through Celite and concentrated *in vacuo* to give the required aniline as a solid.
¹H NMR (DMSO): δ5.59 (2H, bs), 6.64 (2H, d), 7.28 (3H, m), 7.37 (1H, t), 7.52 (1H, s), 7.65 (1H, d), and 12.32 (1H, bs).MS (APCI+ve): [M⁺H]⁺ at m/z 246 (C₁₃H₁₁NO₂S requires [M+H]⁺ at m/z 246).

The example A981 was prepared according to the methods disclosed herein, with particular reference to compounds 1 to 22 above.

Example A981 is 3-(3-chloro-4-hydroxyphenylamino)-4-(2-nitrophenyl)-1H-pyrrole-2,5-dione ([M+H]⁺ : 4071409).

## Claims

1. 3-(3-chloro-4-hydroxyphenylamino)-4-(2-nitrophenyl)-1*H*-pyrrole-2,5-dione or a pharmaceutically acceptable salt or solvate thereof.

2. A pharmaceutical composition comprising 3-(3-chloro-4-hydroxyphenylamino)-4-(2-nitrophenyl)-1*H*-pyrrole-2,5-dione or a pharmaceutically acceptable salt or solvate thereof and a pharmaceutically acceptable carrier.

3. Use of 3-(3-chloro-4-hydroxyphenylamino)-4-(2-nitrophenyl)-1*H*-pyrrole-2,5-dione or a pharmaceutically acceptable salt or solvate thereof in the manufacture of a medicament for the treatment of conditions associated with a need for inhibition of glycogen synthase kinase-3.

4. Use according to claim 3 in the manufacture of a medicament for the treatment of diabetes.

5. Use according to claim 4 in which the diabetes is Type 2 diabetes.

6. Use according to claim 3 in the manufacture of a medicament for the treatment of dementia.

7. Use according to claim 6 in which the dementia is Alzheimer's disease.

8. Use according to claim 6 in which the dementia is manic depression.

9. Use according to claim 3 in the manufacture of a medicament for the treatment of cancer.

## Patentansprüche

1. 3-(3-Chlor-4-hydroxyphenylamino)-4-(2-nitrophenyl)-1*H*-pyrrol-2,5-dion oder ein pharmazeutisch verträgliches Salz oder Solvat davon.

2. Arzneimittel umfassend 3-(3-Chlor-4-hydroxyphenylamino)-4-(2-nitrophenyl)-1*H*pyrrol-2,5-dion oder ein pharmazeutisch verträgliches Salz oder Solvat davon und einen pharmazeutisch verträglichen Träger.

3. Verwendung von 3-(3-Chlor-4-hydroxyphenylamino)-4-(2-nitrophenyl)-1*H*-pyrrol-2,5-dion oder eines pharmazeutisch verträglichen Salzes oder Solvats davon bei der Herstellung eines Medikaments zur Behandlung von Zuständen, die mit einer Notwendigkeit der Hemmung von Glycogensynthasekinase-3 einhergehen.

4. Verwendung nach Anspruch 3 bei der Herstellung eines Medikaments zur Behandlung von Diabetes.

5. Verwendung nach Anspruch 4, wobei der Diabetes ein Diabetes vom Typ II ist.

6. Verwendung nach Anspruch 3 bei der Herstellung eines Medikaments zur Behandlung von Demenz.

7. Verwendung nach Anspruch 6, wobei die Demenz Alzheimer-Krankheit ist.

8. Verwendung nach Anspruch 6, wobei die Demenz manische Depression ist.

9. Verwendung nach Anspruch 3 bei der Herstellung eines Medikaments zur Behandlung von Krebs.

## Revendications

1. 3-(3-chloro-4-hydroxyphénylamino)-4-(2-nitrophényl)-1*H*-pyrrole-2,5-dione ou un de ses sels ou produits de solvatation pharmaceutiquement acceptables.

2. Composition pharmaceutique comprenant de la 3-(3-chloro-4-hydroxyphénylamino)-4-(2-nitrophényl)-1*H*-pyrrole-2,5-dione ou un de ses sels ou produits de solvatation pharmaceutiquement acceptables et un support pharmaceutiquement acceptable.

3. Utilisation de 3-(3-chloro-4-hydroxyphénylamino)-4-(2-nitrophényl)-1*H*-pyrrole-2,5-dione ou d'un de ses sels ou produits de solvatation pharmaceutiquement acceptables dans la production d'un médicament destiné au traitement d'affections associées à un besoin d'inhibition de la glycogène-synthase-kinase-3.

4. Utilisation suivant la revendication 3, dans la production d'un médicament pour le traitement du diabète.

5. Utilisation suivant la revendication 4, dans laquelle le diabète est le diabète de type 2.

6. Utilisation suivant la revendication 3, dans la production d'un médicament destiné au traitement de la démence.

7. Utilisation suivant la revendication 6, dans laquelle la démence est la maladie d'Alzheimer.

8. Utilisation suivant la revendication 6, dans laquelle la démence est la dépression maniaque.

9. Utilisation suivant la revendication 3, dans la production d'un médicament destiné au traitement du cancer.
